# EUROPEAN PATENT APPLICATION

(11) **EP 1 211 235 A2**
(43) Date of publication of application: **05.06.2002**
(21) Application number: 01128333.0
(22) Date of filing: 29.11.2001
(51) Int. Cl.: C07C 51/29, C07C 231/10, C07C 67/36, C07C 45/46, C07C 49/84, C07C 65/24, C07C 69/92

(54) **Preparation process of 4,4'-Dicarboxydiphenyl ethers or derivatives thereof**

(30) Priority: 30.11.2000 JP 2000365904; 30.11.2000 JP 2000365905
(71) Applicant: Adchemco Corporation, Tokyo 102-0073 (JP)
(72) Inventor: Mori, Hiroaki, Chiyoda-ku, Tokyo 102-0073 (JP); Ishihara, Kayo, Chiyoda-ku, Tokyo 102-0073 (JP); Hachiya, Tetsuo, Chiyoda-ku, Tokyo 102-0073 (JP); Kitahara, Makoto, Yokohama-shi, Kanagawa 225-0001 (JP)
(74) Representative: Wächtershäuser, Günter, Prof. Dr.

(57) **Abstract**

4,4'-Dicarboxydiphenyl ether can be prepared by reacting diphenyl ether with an acetylating agent in the presence of a transition metal salt of trifluoromethane-sulfonic acid and an alkali metal perhalogenate to obtain 4,4'-diacetyldiphenyl ether and oxidizing the 4,4'-diacetyldiphenyl ether with an alkali metal hypohalogenite. A 4,4'-dicarboxydiphenyl ether, which is represented by the following formula (2): 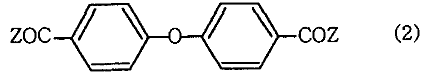 wherein Zs each represents -OH, -OR, -NH₂, -NHR or -NR₂, each R being an alkyl group having a carbon number not greater than 4, or a derivative thereof can be prepared by reacting a 4,4'-dihalodiphenyl ether, which is represented by the following formula (1): 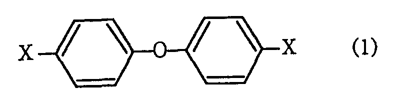 wherein X represents a halogen element, with carbon monoxide, a base and a nucleophile in the presence of a palladium catalyst and a bidentate phosphine compound.

## Description

This invention relates to a preparation process of 4,4'-dicarboxydiphenyl ethers or derivatives thereof.

Various 4,4'-Dicarboxydiphenyl ethers and derivatives thereof have been known to date and have found utility, for example, as raw materials for polyesters and polyamides. Conventionally-known preparation processes of such 4,4'-dicarboxydiphenyl ethers include the following processes:
(1) 4,4'-Dibromodiphenyl ether is reacted with copper cyanide to form a dinitrile [Bull. Soc. Chim. France, **4**, 1285 (1996)], followed by hydrolysis in a manner known *per se* in the art. This process, however, has to use toxic copper cyanide so that it involves problems in environment and handling safety.
(2) Diphenyl ether is dichloromethylated, followed by hydrolysis with potassium permanganate [J. Chem. Soc., 1604 (1961)]. This process is, however, not practical because the yield of the dicholomethylation of diphenyl ether is low.
(3) Diphenyl ether is reacted with (R)₂NCOCl in the presence of anhydrous aluminum chloride, followed by hydrolysis [Zn. Org. Chem. **3**(1), 46 (1967)]. The use of anhydrous aluminum chloride, however, causes troubles in reaction post-treatment, waste water treatment and the like.
(4) A 4,4'-dialkylphenyl ether is oxidized with molecular oxygen in the presence of a transition metal catalyst (JP 63-310846 A or the like) . This process, however, is accompanied by problems in that the overall yield through the dialkylation and oxidation steps is low and the oxidation reaction is a potential safety hazard.
(5) 4-Nitrobenzonitrile is condensed into 4,4'-dicyanodiphenyl ether in the presence of an alkali metal nitrite and an alkali metal carbonate, followed by hydrolysis (Chinese Patent No. 1052300). This problem, however, involves a problem in economy because 4-nitrobenzonitrile is costly.
(6) A 4,4'-dihalophenyl ether is reacted with carbon monoxide, a base and an alcohol in the presence of a transition metal compound as a catalyst to obtain a 4,4'-dipheny ether dicarboxylic acid dialkyl ester, followed by the hydrolysis of the ester in a manner known *per se* in the art. This process, however, cannot obtain the target compound with satisfactory reaction performance unless the pressure of carbon monoxide is raised generally to a pressure as high as 5 to 10 MPa upon reacting the 4,4'-dihalophenyl ether with carbon monoxide, the base and the alcohol in the presence of the transition metal compound as the catalyst. This process, therefore, involves limitations to production facilities and problems in safety.

An object of the present invention is, therefore, to provide an industrially advantageous process for the preparation of a 4,4'-dicarboxydiphenyl ether or a derivative thereof, the industrially advantageous preparation of which has not been satisfactorily achieved by any of the conventional processes.

The above-described object can be achieved by the invention to be described hereinafter.

In a first aspect of the present invention, there is thus provided a process for the preparation of 4,4'-dicarboxydiphenyl ether, characterized by the following steps:
reacting diphenyl ether with an acetylating agent in the presence of a transition metal salt of trifluoromethanesulfonic acid and an alkali metal perhalogenate to obtain 4,4'-diacetyl-diphenyl ether; and
oxidizing the 4,4'-diacetyldiphenyl ether with an alkali metal hypohalogenite.

In a second aspect of the present invention, there is also provided a process for the preparation of a 4,4'-dicarboxydiphenyl ether represented by the following formula (2): 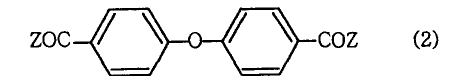 wherein Zs each represents -OH, -OR, -NH₂, -NHR or -NR₂, each R being an alkyl group having a carbon number not greater than 4, or a derivative thereof, characterized by:
reacting a 4,4'-dihalodiphenyl ether, which is represented by the following formula (1): 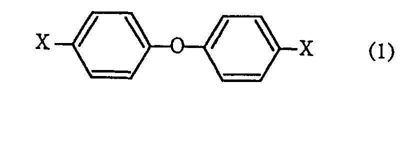
wherein X represents a halogen element, with carbon monoxide, a base and a nucleophile in the presence of a palladium catalyst and a bidentate phosphine compound.

According to the first aspect of the present invention, 4,4'-dicarboxydiphenyl ether can be prepared in an industrial scale with excellent economy and safety while solving the various problems of the conventional processes.

According to the second aspect of the present invention, on the other hand, the 4,4'-dicarboxydiphenyl ether or its derivative can be prepared in an industrial scale with excellent economy without using aluminum chloride.

The present invention will hereinafter be described in further detail based on certain preferred embodiments.

### [First Aspect of the Invention]

Using structural formulas, the reaction scheme of the preparation process according to the first aspect of the present invention can be expressed as follows:

### First Step

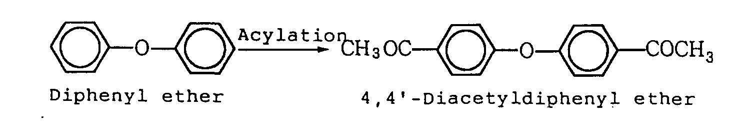

### Second Step

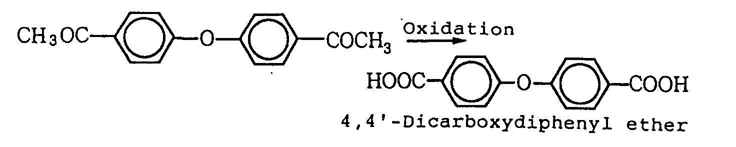

With a view to solving the conventional problems, the present inventors have proceeded with an extensive investigation. As a result, it has been found that a reaction of an acetylating agent such as acetic anhydride with diphenyl ether in the presence of a transition metal salt, such as hafnium trifluoromethanesulfonate, and an alkali metal perhalogenate, such as sodium perchlorate, can form a diacetyl derivative with a high yield and also that a subsequent haloform reaction with an alkali metal hypohalogenite can yield 4,4'-dicarboxydiphenyl ether.

Illustrative of the transition metal in the transition metal salt of trifluoromethanesulfonic acid, said salt being employed in the present invention, are those belonging to Group 3A or 4A of the Periodic Table, in the notation as defined in *Chemical Handbook*, Basic, 4^{th} Edition (Revised, 1993)(edited by the Chemical Society of Japan; published by Maruzen Co., Lt.), inside front cover, for example, scandium, yttrium, lanthanum, cerium, praseodymium, neodymium, samarium, europium, gadolinium, terbium, dysprosium, holmium, erbium, thulium, ytterbium, lutetium and hafnium. In particular, yttrium trifluoromethanesulfonate, lanthanum trifluoromethanesulfonate and hafnium trifluoro-methanesulfonate are preferred.

The transition metal salt of trifluoromethanesulfonic acid may be used preferably in a proportion of from 0.01 to 1 mole per mole of diphenyl ether, with 0.05 to 0.5 mole being more preferred. The transition metal salt of trifluoromethanesulfonic acid is costly. From the economical consideration, it is therefore not preferred to discard the metal salt after using it only once. The transition metal salt of trifluoromethanesulfonic acid can be reused in the subsequent reaction by quantitatively recovering it from a residue after separation of 4,4'-diacetyldiphenyl ether as an intermediate from the reaction mixture.

As the alkali metal perhalogenate for use in this reaction, use of lithium perchlorate is particularly preferred. The alkali metal perhalogenate may be used preferably in a proportion of from 1 to 50 moles per mole of diphenyl ether, with 5 to 20 moles being more preferred.

Usable examples of the acetylating agent for use in the present invention can include acetic acid, acetic anhydride and acetyl chloride, with acetic anhydride being particularly preferred. The acetylating agent may be used preferably in a proportion of from 2 to 20 moles per mole of diphenyl ether, with 4 to 10 moles being more preferred.

The acylation in the first step may preferably be conducted in a solvent. In general, it is possible to use any one of solvents which are typically employed upon acetylation of aromatic compounds. Usable examples can include organic halogen compounds such as carbon disulfide and dichloromethane, and nitro compounds such as nitromethane and nitrobenzene. However, use of a nitro compound such as nitromethane or nitrobenzene is preferred.

Preferably, the above-described reaction is conducted in a nitrogen gas atmosphere. The reaction can be effected in a temperature range of from 0 to 100°C, and at a temperature of from 20 to 50°C, better results are available. A reaction temperature higher than 100°C is not preferred, because such an unduly high temperature leads to a reduction in yield due to side reactions. 4,4'-Diacetyldiphenyl ether is recovered from the reaction mixture by distillation under reduced pressure. This 4,4'-diacetyldiphenyl ether can be converted into 4,4'-dicarboxydiphenyl ether by using the haloform reaction.

The alkali metal hypohalogenite as an oxidizing agent may be formed in the reaction system by adding a halogen to a caustic alkali metal. As an alternative, a commercial alkali metal hypohalogenite can also be used without any problem. It is particularly preferred to use sodium hypochlorite as the alkali metal hypohalogenite. The oxidizing reaction in the second step can be effected preferably at a temperature of from 20 to 60°C. After the reaction, the reaction product, 4,4'-dicarboxy-diphenyl ether can be recovered, for example, as will be described next. Sodium hydrogensulfite is added to deactivate any excess alkali metal hypohalogenite. The reaction mixture is acidified with hydrochloric acid. The resulting precipitate is collected by filtration and then washed with water to obtain 4,4'-dicarboxydiphenyl ether.

### [Second Aspect of the Present Invention]

With a view to solving the above-described conventional problems, the present inventors also proceeded with another extensive investigation. As a result, it has been found that upon reacting a 4,4'-dihalodiphenyl ether with carbon monoxide, a base and a nucleophile in the presence of a palladium catalyst, use of a bidentate phosphine compound as a ligand for the palladium catalyst makes it possible to prepare 4,4'-dicarboxydiphenyl ether or a derivative thereof at a carbon monoxide pressure much lower than that needed conventionally with reaction performance surpassing those of the conventional processes.

As the 4,4'dihalodiphenyl ether for use in the present invention, 4,4'-difluorodiphenyl ether, 4,4'-dichlorophenyl ether, 4,4'-dibromodiphenyl ether and 4,4'-diiodophenyl ether are all usable. In view of reactivity and raw material cost, however, use of 4,4'-dibromodiphenyl ether is preferred.

Any base can be used in the present invention insofar as it can scavenge hydrogen halide, which is formed in the reaction, by neutralization. Examples can include aliphatic and aromatic amines, quaternary ammonium hydroxide, alcoholates, alkali metal hydroxides, alkaline earth hydroxides, alkali metal carbonates, and strongly basic ion-exchange resins. Preferred are aliphatic tertiary amines, for example, triethylamine and tributylamine. Theoretically, the base may be used in a proportion equivalent to or greater than the hydrogen halide which is to be formed in the reaction. Preferably, however, the base can be used in a proportion of from 1.5 to 5 times the hydrogen halide in terms of equivalents.

Examples of the palladium catalyst for use in the present invention can include palladium(II) halides such as palladium(II) chloride, palladium(II) bromide and palladium (II) iodide; the palladium(II) salts of organic acids, such as palladium(II) acetate and palladium(II) propionate; and divalent palladium compounds such as palladium(II) acetylacetonate, palladium(II) cyanide, palladium(II) nitrate and palladium(II) sulfate.

The palladium catalyst may be used in a proportion of from 0.001 to 10 mol%, preferably 0.005 to 2 mol% based on the 4,4'-dihalodiphenyl ether. Use of the palladium catalyst in an unduly small proportion leads to a reduction in yield, while use of the palladium catalyst in an excessively large proportion is economically unjustifiable because the palladium catalyst is costly.

Examples of the bidentate phosphine compound for use in the present invention can include bidentate phosphines such as 1,2-bis(diphenylphosphino)ethane, 1,3-bis(diphenylphosphino)propane and 1,4-bis(diphenylphosphino)butane. This phosphine ligand may be used preferably in a molar proportion of from 0.5 to 10 times as much as the palladium catalyst, with a range of 1 to 4 times being more preferred.

In the present invention, the resulting 4,4'-disubstituted diphenyl ether differs depending upon the nucleophile used. Usable examples of the nucleophile can include water; alcohols such as methanol, ethanol and phenol; and amines such as triethylamine and tributyl amine. Use of water as the nucleophile results in the formation of 4,4'-diphenylether dicarboxylic acid, use of an alcohol gives the corresponding diester of 4,4'-diphenylether dicarboxylic acid, and use of an amine yields the corresponding diamide of 4,4'-diphenylether dicarboxylic acid. Selection of a desired nuleophile makes it possible to directly synthesize a target polymer raw material. No particular limitation is imposed on the amount of the nucleophile to be used, insofar as it is equal to or greater than the amount theoretically required to form the 4,4'-di-substituted diphenyl ether.

It is preferred to use a solvent in the present invention. Usable examples can include aromatic hydrocarbons such as benzene and toluene; aprotic solvents such as dimethylformamide, dimethyl sulfoxide and acetonitrile; and ether-type solvents such as dioxane and tetrahydrofuran.

The reaction can be conducted in a carbon monoxide atmosphere under environmental or elevated pressure. When the reaction is carried out under elevated pressure, an autoclave can be used, and the pressure can be set preferably at 0 to 2 MPa, more preferably at 0.1 to 1 MPa, both in terms of gauge pressure. Although the reaction proceeds without any problem even at a pressure higher than 2MPa, such an excessively high pressure is not preferred in view of limitations to production facilities and safety problem in high-pressure reactions.

The reaction can be conducted in a temperature range of from 30 to 200°C, and at 80 to 150°C, better results are available. A reaction temperature higher than 200°C is not preferred as such an excessively high temperature leads to a reduction in the yield of the target compound due to side reactions.

The present invention will next be described in further detail based on Examples. It should, however, be borne in mind that the present invention is by no means limited by or to the following Examples.

### [First Aspect of the Invention]

### Example 1

The interior of a 200-mL flask fitted with a stirrer and a thermometer was converted into a nitrogen gas atmosphere. Diphenyl ether (3.4 g, 21 mmol), acetic anhydride (8.2 g, 80 mmol), hafnium trifluoromethanesulfonate (1.6 g, 2.1 mmol), lithium perchlorate (25.4 g, 0.239 mol) and nitromethane (40 mL) were charged, followed by a reaction at 25°C for 8 hours. The reaction mixture was distilled under reduced pressure to yield 4,4'diacetyldiphenyl ether.

The thus-obtained diacetyl derivative, sodium hypochlorite (110 g; effective chlorine content: 5 wt.%) and sodium hydroxide (6.2 g, 0.16 mol) were then charged into a 200-mL flask fitted with a stirrer and a reflux condenser, and were reacted at 55°C until the reaction mixture change from a slurry into a homogeneous solution. After completion of the reaction, 40 wt.% sodium hydrogensulfite (50 g) was added, followed by the addition of concentrated hydrochloric acid to acidify the reaction mixture. The resulting precipitate was collected by filtration, washed with a sufficient amount of water, and then dried to afford 4,4'-dicarboxydiphenyl ether (4.3 g; yield: 79.5% based on the diphenyl ether).

### Example 2

The interior of a 200-mL flask fitted with a stirrer and a thermometer was converted into a nitrogen gas atmosphere. Diphenyl ether (3.4 g, 21 mmol), acetic anhydride (8.2 g, 80 mmol), hafnium trifluoromethanesulfonate (1.6 g, 2.1 mmol), lithium perchlorate (25.4 g, 0.239 mol), nitrobenzene (10 mL) and nitromethane (30 mL) were charged, followed by a reaction at 25°C for 8 hours. The reaction mixture was distilled under reduced pressure to yield 4,4'diacetyldiphenyl ether.

The thus-obtained diacetyl derivative, sodium hypochlorite (110 g; effective chlorine content: 5 wt.%) and sodium hydroxide (6.2 g, 0.16 mol) were then charged into a 200-mL flask fitted with a stirrer and a reflux condenser, and were reacted at 55°C until the reaction mixture change from a slurry into a homogeneous solution. After completion of the reaction, 40 wt.% sodium hydrogensulfite (50 g) was added, followed by the addition of concentrated hydrochloric acid to acidify the reaction mixture. The resulting precipitate was collected by filtration, washed with a sufficient amount of water, and then dried to afford 4,4'-dicarboxydiphenyl ether (4.0 g; yield: 73.2% based on the diphenyl ether).

### Example 3

The interior of a 200-mL flask fitted with a stirrer and a thermometer was converted into a nitrogen gas atmosphere. Diphenyl ether (3.4 g, 21 mmol), acetic anhydride (8.2 g, 80 mmol), yttrium trifluoromethanesulfonate (1.1 g, 2.1 mmol), lithium perchlorate (25.4 g, 0.239 mol) and nitromethane (40 mL) were charged, followed by a reaction at 25°C for 8 hours. The reaction mixture was distilled under reduced pressure to yield 4,4'diacetyldiphenyl ether.

The thus-obtained diacetyl derivative, sodium hypochlorite (110 g; effective chlorine content: 5 wt.%) and sodium hydroxide (6.2 g, 0.16 mol) were then charged into a 200-mL flask fitted with a stirrer and a reflux condenser, and were reacted at 55°C until the reaction mixture change from a slurry into a homogeneous solution. After completion of the reaction, 40 wt.% sodium hydrogensulfite (50 g) was added, followed by the addition of concentrated hydrochloric acid to acidify the reaction mixture. The resulting precipitate was collected by filtration, washed with a sufficient amount of water, and then dried to afford 4,4'-dicarboxydiphenyl ether (3.7 g; yield: 68.4% based on the diphenyl ether).

### Example 4

The interior of a 200-mL flask fitted with a stirrer and a thermometer was converted into a nitrogen gas atmosphere. Diphenyl ether (3.4 g, 21 mmol), acetic anhydride (8.2 g, 80 mmol), hafnium trifluoromethanesulfonate (0.8 g, 1.0 mmol), lithium perchlorate (25.4 g, 0.239 mol) and nitromethane (40 mL) were charged, followed by a reaction at 25°C for 8 hours. The reaction mixture was distilled under reduced pressure to yield 4,4'diacetyldiphenyl ether.

The thus-obtained diacetyl derivative, sodium hypochlorite (110 g; effective chlorine content: 5 wt.%) and sodium hydroxide (6.2 g, 0.16 mol) were then charged into a 200-mL flask fitted with a stirrer and a reflux condenser, and were reacted at 55°C until the reaction mixture change from a slurry into a homogeneous solution. After completion of the reaction, 40 wt.% sodium hydrogensulfite (50 g) was added, followed by the addition of concentrated hydrochloric acid to acidify the reaction mixture. The resulting precipitate was collected by filtration, washed with a sufficient amount of water, and then dried to afford 4,4'-dicarboxydiphenyl ether (4.3 g; yield: 79.5% based on the diphenyl ether).

### Example 5

From the reaction mixture from which the 4,4'-diacetyldiphenyl ether had been obtained in Example 1, hafnium trifluoromethanesulfonate was recovered. As a result, hafnium trifluoromethanesulfonate was recovered in an amount as much as 90 wt.% based on its initial charge. Fresh hafnium trifluoromethanesulfonate was added to the recovered hafnium trifluoromethanesulfonate to give a total amount of 3.4 g. Using the thus-obtained hafnium trifluoromethanesulfonate as a catalyst, the procedures of Example 1 were repeated. The results were similar to those obtained in Example 1.

### [Second Aspect of the Invention]

### Example 1

Charged into a 200-mL autoclave fitted with a thermometer and a stirrer were 4,4'-dibromodiphenyl ether (8.2 g, 25 mmol), triethylamine (6.1 g, 60 mmol), palladium chloride (44.3 mg, 0.25 mmol), 1,4-bis(diphenylphosphino)butane (54.4 mg, 0.125 mmol), ethanol (20 mL) and toluene (20 mL). Carbon monoxide was charged to a gauge pressure of 1 MPa, and the contents were heated to conduct a reaction at 120°C for 10 hours under the same pressure. After the reaction, the reaction mixture was allowed to cool down to room temperature, the precipitated triethylamine hydrcchloride was separated by filtration and then washed with toluene. The filtrate and the washing were combined to obtain an organic layer. The organic layer was washed with water and then dried over anhydrous sodium sulfate. The solvent was distilled off to afford 4,4'-diphenyl ether dicarboxylic acid diethyl ester (7.5 g; yield: 95.5% based on the 4,4'-dibromodiphenyl ether).

### Example 2

Charged into a 200-mL autoclave fitted with a thermometer and a stirrer were 4,4'-dibromodiphenyl ether (8.2 g, 25 mmol), triethylamine (6.1 g, 60 mmol), palladium chloride (44.3 mg, 0.25 mmol), 1,4-bis(diphenylphosphino)butane (54.4 mg, 0.125 mmol), methanol (20 mL) and toluene (20 mL). Carbon monoxide was charged to a gauge pressure of 1 MPa, and the contents were heated to conduct a reaction at 120°C for 10 hours under the same pressure. After the reaction, the reaction mixture was allowed to cool down to room temperature, the precipitated triethylamine hydrochloride was separated by filtration and then washed with toluene. The filtrate and the washing were combined to obtain an organic layer. The organic layer was washed with water and then dried over anhydrous sodium sulfate. The solvent was distilled off to afford 4,4'-diphenylether dicarboxylic acid dimethyl ether (7.2 g; yield: 95.0% based on the 4,4'-dibromodiphenyl ether).

### Example 3

Charged into a 200-mL autoclave fitted with a thermometer and a stirrer were 4,4'-dibromodiphenyl ether (8.2 g, 25 mmol), triethylamine (6.1 g, 60 mmol), palladium chloride (44.3 mg, 0.25 mmol), 1,2-bis(diphenylphosphino)ethane (49.8 mg, 0.125 mmol), ethanol (20 mL) and toluene (20 mL). Carbon monoxide was charged to a gauge pressure of 1 MPa, and the contents were heated to conduct a reaction at 120°C for 10 hours under the same pressure. After the reaction, the reaction mixture was allowed to cool down to room temperature, the precipitated triethylamine hydrochloride was separated by filtration and then washed with toluene. The filtrate and the washing were combined to obtain an organic layer. The organic layer was washed with water and then dried over anhydrous sodium sulfate. The solvent was distilled off to afford 4,4'-diphenylether dicarboxylic acid diethyl ester (7.4 g; yield: 93.6% based on the 4,4'-dibromodiphenyl ether).

### Example 4

Charged into a 200-mL autoclave fitted with a thermometer and a stirrer were 4,4'-dibromodiphenyl ether (8.2 g, 25 mmol), triethylamine (6.1 g, 60 mmol), palladium chloride (44.3 mg, 0.25 mmol), 1,4-bis(diphenylphosphino)butane (54.4 mg, 0.125 mmol), ethanol (20 mL) and toluene (20 mL). Carbon monoxide was charged to a gauge pressure of 1 MPa, and the contents were heated to conduct a reaction at 150°C for 6 hours under the same pressure. After the reaction, the reaction mixture was allowed to cool down to room temperature, the precipitated triethylamine hydrochloride was separated by filtration and then washed with toluene. The filtrate and the washing were combined to obtain an organic layer. The organic layer was washed with water and then dried over anhydrous sodium sulfate. The solvent was distilled off to afford 4,4'-diphenylether dicarboxylic acid diethyl ester (7.4 g; yield: 93.7% based on the 4,4'-dibromodiphenyl ether).

### Example 5

Charged into a 200-mL autoclave fitted with a thermometer and a stirrer were 4,4'-dibromodiphenyl ether (8.2 g, 25 mmol), triethylamine (6.1 g, 60 mmol), palladium chloride (44.3 mg, 0.25 mmol), 1,4-bis(diphenylphosphino)butane (54.4 mg, 0.125 mmol), ethanol (20 mL) and toluene (20 mL). Carbon monoxide was charged to a gauge pressure of 0.8 MPa, and the contents were heated to conduct a reaction at 150°C for 10 hours under the same pressure. After the reaction, the reaction mixture was allowed to cool down to room temperature, the precipitated triethylamine hydrochloride was separated by filtration and then washed with toluene. The filtrate and the washing were combined to obtain an organic layer. The organic layer was washed with water and then dried over anhydrous sodium sulfate. The solvent was distilled off to afford 4,4'-diphenylether dicarboxylic acid diethyl ester (7.5 g; yield: 95.4% based on the 4,4'-dibromodiphenyl ether).

### Example 6

Charged into a 200-mL autoclave fitted with a thermometer and a stirrer were 4,4'-dibromodiphenyl ether (8.2 g, 25 mmol), triethylamine (6.1 g, 60 mmol), palladium chloride (44.3 mg, 0.25 mmol), 1,4-bis(diphenylphosphino)butane (54.4 mg, 0.125 mmol), water (20 mL) and acetonitrile (20 mL). Carbon monoxide was charged to a gauge pressure of 1 MPa, and the contents were heated to conduct a reaction at 120°C for 10 hours under the same pressure. After the reaction, the reaction mixture was allowed to cool down to room temperature, and water (100 mL) was added to the reaction mixture to dilute the same. Insoluble matter was filtered off, and the filtrate was washed with toluene. Concentrated hydrochloric acid was added to the water phase to acidify the same such that a carboxylic acid compound was caused to precipitate. The carboxylic acid compound was collected by filtration, washed with dilute hydrochloric acid, and then dried to afford 4,4'-diphenylether dicarboxylic acid (6.0 g; yield: 92.6% based on the 4,4'-dibromodiphenyl ether) .

### Example 7

Charged into a 200-mL autoclave fitted with a thermometer and a stirrer were 4,4'-dibromodiphenyl ether (8.2 g, 25 mmol), triethylamine (6.1 g, 60 mmol), palladium chloride (22.2 mg, 0.125 mmol), 1,4-bis(diphenylphosphino)butane (27.2 mg, 0.062 mmol), ethanol (20 mL) and toluene (20 mL). Carbon monoxide was charged to a gauge pressure of 1 MPa, and the contents were heated to conduct a reaction at 150°C for 15 hours under the same pressure. After the reaction, the reaction mixture was allowed to cool down to room temperature, the precipitated triethylamine hydrobromide was separated by filtration and then washed with toluene. The filtrate and the washing were combined to obtain an organic layer. The organic layer was washed with water and then dried over anhydrous sodium sulfate. The solvent was distilled off to afford 4,4'-diphenylether dicarboxylic acid diethyl ester (7.4 g; yield: 94.1% based on the 4,4'-dibromodiphenyl ether).

### Example 8

Charged into a 200-mL autoclave fitted with a thermometer and a stirrer were 4, 4'-dibromodiphenyl ether (8.2 g, 25 mmol), diethylamine (8.8 g, 0.12 mol), palladium chloride (44.3 mg, 0.25 mmol), 1,4-bis(diphenylphosphino)butane (54.4 mg, 0.125 mmol) and toluene (20 mL). Carbon monoxide was charged to a gauge pressure of 1 MPa, and the contents were heated to conduct a reaction at 120°C for 10 hours under the same pressure. The precipitated diethylamine hydrobromide was separated by filtration and then washed with toluene. The filtrate and the washing were combined to obtain an organic layer. The organic layer was washed with water and then dried over anhydrous sodium sulfate. The solvent was distilled off to afford 4, 4'-diphenyl ether di-N,N-diethylamide (6.4 g; yield: 90.2% based on the 4,4'-dibromodiphenyl ether).

## Claims

1. A process for the preparation of 4,4'-dicarboxydiphenyl ether, **characterized by** the following steps:
reacting diphenyl ether with an acetylating agent in the presence of a transition metal salt of trifluoromethanesulfonic acid and an alkali metal perhalogenate to obtain 4,4'-diacetyl-diphenyl ether; and
oxidizing said 4,4'-diacetyldiphenyl ether with an alkali metal hypohalogenite.

2. A preparation process according to claim 1, wherein a transition metal in said transition metal salt of trifluromethanesulfonic acid is a metal belonging to Group 3A or 4A of the Periodic Table.

3. A preparation process according to claim 2, wherein said transition metal is selected from yttrium, lanthanum or hafnium.

4. A preparation process according to claim 1, wherein said transition metal salt of trifluoromethanesulfonic acid is used in a proportion of from 0.01 to 1 mole per mole of diphenyl ether.

5. A preparation process according to claim 1, wherein said transition metal salt of trifluoromethanesulfonic acid is repeatedly reused.

6. A preparation process according to claim 1, wherein said alkali perhalogenate is lithium perchlorate, and lithium perchlorate is used in a proportion of from 1 to 50 moles per mole of diphenyl ether.

7. A preparation process according to claim 1, wherein said acetylating agent is acetic anhydride, and acetic anhydride is used in a proportion of from 2 to 20 moles per mole of diphenyl ether.

8. A process for the preparation of a 4,4'-dicarboxydiphenyl ether represented by the following formula (2) : 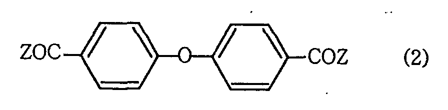 wherein Zs each represents -OH, -OR, -NH₂, -NHR or -NR₂, each R being an alkyl group having a carbon number not greater than 4, or a derivative thereof, **characterized by**:
reacting a 4,4'-dihalodiphenyl ether, which is represented by the following formula (1): 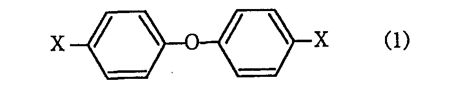
wherein X represents a halogen element, with carbon monoxide, a base and a nucleophile in the presence of a palladium catalyst and a bidentate phcsphine compound.

9. A preparation process according to claim 8, wherein said reaction is conducted at a gauge pressure of from 0 to 2 MPa.

10. A preparation process according to claim 8, wherein said 4,4'-dihalodiphenyl ether is 4,4'-dibromodiphenyl ether.

11. A preparation process according to claim 8, wherein said base is an aliphatic tertiary amine.

12. A preparation process according to claim 8, wherein said palladium catalyst is used in a proportion of from 0.001 to 10 mol% based on said 4,4'-dihalodiphenyl ether.

13. A preparation process according to claim 8, wherein said bidentate phosphine compound is used in a molar proportion of from 0.5 to 10 times as much as said palladium catalyst.

14. A preparation process according to claim 8, wherein said nucleophile is water, an alcohol having 1 to 4 carbon atoms, ammonia, or a mono-, di- or trialkylamine having 1 to 4 carbon atoms.

15. Use of a transition metal salt of trifluoromethanesulfonic acid and an alkali metal perhalogenate in the preparation of 4,4'-diacetyldiphenyl ether

16. Use of a bidentate phosphine compound as a ligand for a palladium catalyst in the preparation of a 4,4'-dihalodiphenyl ether or a derivative thereof.
